# EUROPEAN PATENT APPLICATION

(11) **EP 4 847 612 A1**
(43) Date of publication of application: **02.12.2026**
(21) Application number: 25744672.4
(22) Date of filing: 23.01.2025
(51) Int. Cl.: C12N 9/90, C12N 15/61, C12N 1/21, C12N 15/70, C12P 19/02, C12P 19/24, C12R 1/19

(54) **ALDOHEXOSE-2-EPIMERASE OR MUTANT THEREOF AND USE THEREOF**

(30) Priority: 25.01.2024 CN 202410107470; 06.03.2024 CN 202410256912; 20.03.2024 CN 202410323978
(71) Applicant: Beijing Chengzhi Life Science Co., Ltd., Beijing 10084 (CN); Chengzhi Life Science Co., Ltd., Beijing 100084 (CN)
(72) Inventor: JIA, Minze, Beijing 100084 (CN); DONG, Gang, Beijing 100084 (CN); GAO, Runxiang, Beijing 100084 (CN); CAI, Xia, Beijing 100084 (CN); YAO, Hongwei, Beijing 100084 (CN)
(74) Representative: Lavoix
(86) International application number: PCT/CN2025/074227
(87) International publication number: WO 2025/157209

(57) **Abstract**

Provided are an aldohexose-2-epimerase or a mutant thereof and a use thereof. The aldohexose-2-epimerase comprises a sequence of any one of SEQ ID NOs: 1-5 or a sequence having at least 90% identity to the sequence of any one of SEQ ID NOs: 1-5, or the aldohexose-2-epimerase mutant comprises one or more mutations based on a reference sequence, wherein the amino acid sequence of the reference sequence is as shown in SEQ ID NO: 5. The aldohexose-2-epimerase or the mutant thereof can convert aldohexose, for example, converting D-glucose into D-mannose or D-mannose into D-glucose. Due to high specificity and good stability, the aldohexose-2-epimerase or the mutant thereof can convert the relatively inexpensive raw material D-glucose into D-mannose, significantly reducing the production cost of D-mannose while achieving green production with "emission reduction and energy savings".

## Description

### TECHNICAL FIELD

The present application relates to the field of enzyme engineering technology, and in particular, to an aldohexose-2-epimerase or a mutant thereof and uses thereof.

### BACKGROUND

Functional sugars, as sucrose substitutes, have broad prospects in the fields of food, pharmaceuticals, health care, and the like due to their unique physiological functions. D-mannose is one of the functional sugars, having prebiotic functions, and can also enhance wound healing and avoid bacterial infections. D-mannose can directly bind to the fimbriae of *Escherichia coli, Enterococcus faecalis*, and *Staphylococcus* that cause urinary tract infections, causing them to detach from the urethral wall, thereby effectively reducing the recurrence risk of urinary tract infections.

Currently, the preparation of D-mannose mainly includes chemical synthesis methods and biological enzymatic methods.

The production cost of chemical synthesis methods is relatively high, and the production process is prone to generating a large amount of acidic waste liquid that pollutes the environment. Moreover, reaction by-products make the separation and preparation of high-purity D-mannose relatively difficult. Currently, mannose can be prepared by isomerizing glucose under alkaline conditions, but this method suffers from poor yield and heavy refining load due to the decomposition of sugars. In addition, preparing mannose by isomerizing fructose under alkaline conditions not only faces the same problems of yield and refining load but also has the problem that fructose remains in the reaction system and is difficult to separate from mannose.

Biological enzymatic methods have mild reaction conditions, few by-products, easy separation and purification, no environmental pollutions, and low costs. Therefore, the use of biological enzymatic methods to prepare D-mannose has become a future research trend.

Regarding biological enzymatic methods, it is currently common to use mannose isomerase to act on fructose, aldose-ketose isomerase to act on glucose or fructose, and cellobiose 2-epimerase to act on glucose through side reactions to prepare mannose. However, whether fructose remains as a substrate residue or as a by-product, it will have adverse affects on the subsequent separation and purification of mannose. Therefore, it can be seen that a low-cost and efficient biological enzymatic method for preparing D-mannose needs further development.

### SUMMARY

In view of the technical problems existing in the prior art, the present application provides an aldohexose-2-epimerase or a mutant thereof and uses thereof. The aldohexose-2-epimerase or mutant thereof provided in the present application can convert aldohexoses, for example, can convert D-glucose into D-mannose or convert D-mannose into D-glucose.

The specific technical solutions of the present application are as follows:
1. An aldohexose-2-epimerase or a mutant thereof, wherein the aldohexose-2-epimerase comprises a sequence as set forth in any one of SEQ ID NOs: 1-5 or a sequence having at least 90% identity to the sequence as set forth in any one of SEQ ID NOs: 1-5; or
   the aldohexose-2-epimerase mutant comprises one or more mutations based on a reference sequence, wherein the amino acid sequence of the reference sequence is as set forth in SEQ ID NO: 5.
2. The aldohexose-2-epimerase or a mutant thereof according to item 1, wherein the amino acid sequence of the mutant comprises at least one an amino acid mutation at the positions corresponding to G354 and T355 of SEQ ID NO: 5, preferably comprises amino acid mutations at the positions corresponding to G354 and T355 of SEQ ID NO: 5.
3. An aldohexose-2-epimerase mutant, comprising an amino acid sequence as set forth in any one of SEQ ID NOs: 6-8 or an amino acid sequence having at least 90% identity to the amino acid sequence as set forth in any one of SEQ ID NOs: 6-8.
4. The aldohexose-2-epimerase or a mutant thereof according to any one of items 1-3, wherein the aldohexose is glucose or mannose.
5. A nucleic acid molecule, encoding the aldohexose-2-epimerase or a mutant thereof according to any one of items 1-4.
6. The nucleic acid molecule according to item 5, wherein the nucleic acid molecule comprises a sequence as set forth in any one of SEQ ID NOs: 9-16 or a sequence having at least 90% identity to the sequence as set forth in any one of SEQ ID NOs: 9-16.
7. An expression vector, comprising the nucleic acid molecule according to item 5 or 6.
8. The expression vector according to item 7, wherein the expression vector is a plasmid, a cosmid, a phage, or a viral vector; preferably a plasmid.
9. A host cell, comprising the expression vector according to item 7 or 8;
   preferably, the host cell is a eukaryotic cell, a prokaryotic cell, or a bacterial cell; preferably a prokaryotic cell; more preferably *Escherichia coli.*
10. Use of the aldohexose-2-epimerase or a mutant thereof according to any one of items 1-4 in aldohexose conversion.
11. A method for converting an aldohexose, comprising using the aldohexose-2-epimerase or a mutant thereof according to any one of items 1-4 to catalyze an aldohexose reaction, thereby converting the aldohexose.
12. The method according to item 11, wherein the reaction temperature is 20-55°C, preferably 30-45°C; and/or
   the reaction pH is 6.5-10, preferably 7.5-8.5; and/or
   the reaction time is 0.5-10 hours, preferably 0.5-6 hours.

### Effects of the Application

The aldohexose-2-epimerase or a mutant thereof described in the present application can convert aldohexoses, for example, can convert D-glucose into D-mannose or convert D-mannose into D-glucose. Due to its high specificity and good stability, the relatively inexpensive raw material D-glucose can be converted into D-mannose, significantly reducing the production cost of D-mannose while achieving "emission reduction and consumption reduction" green production.

The aldohexose-2-epimerase described in the present application is used for the conversion between monosaccharides (aldohexoses), i.e., converting D-glucose into D-mannose or converting D-mannose into D-glucose, and the aldohexose-2-epimerase has relatively high activity in converting aldohexoses. Among them, the enzyme activity of using this enzyme to convert D-glucose into D-mannose is about 6 U/mg, which can greatly improve the efficiency of converting D-glucose to produce D-mannose.

Compared with the wild type (aldohexose-2-epimerase), the specific enzyme activity of the mutant for converting D-glucose into D-mannose is increased by about 2.2 times, and under the same conditions, the conversion rate of converting D-glucose into D-mannose is increased by about 3.6 times, reaching 22.6%.

### BRIEF DESCRIPTION OF THE DRAWINGS

Figure 1 is a schematic diagram showing the glucose conversion rate over time when glucose is converted into mannose using aldohexose-2-epimerase.
Figure 2 is a schematic diagram showing the glucose conversion rate as a function of reaction temperature when glucose is converted into mannose using aldohexose-2-epimerase.
Figure 3 is a schematic diagram showing the glucose conversion rate as a function of reaction pH when glucose is converted into mannose using aldohexose-2-epimerase.
Figure 4 is a schematic diagram showing the glucose conversion rate when glucose is converted into mannose using the aldohexose-2-epimerase wild type and mutants.
Figure 5 is a schematic diagram showing the change in glucose conversion rate as a function of reaction time when glucose is converted into mannose using an aldohexose-2-epimerase mutant.

### DETAILED DESCRIPTION

The present application is described in detail below in conjunction with the described embodiments. Although specific embodiments of the present application are shown, it should be understood that the present application may be implemented in various forms and should not be limited to the embodiments set forth herein. Rather, these embodiments are provided so that this application will be more thoroughly understood and will fully convey the scope of the application to those skilled in the art.

It should be noted that certain terms are used in the specification and claims to refer to specific components. Those skilled in the art should understand that technicians may use different terms to refer to the same component. This specification and claims do not use the difference in terms as a way to distinguish components, but rather use the difference in function of components as the criterion for distinction. As mentioned throughout the specification and claims, "comprising" or "including" is an open-ended term, so it should be interpreted as "including but not limited to". The subsequent description of the specification is a preferred embodiment for implementing the present application, but the description is for the purpose of the general principles of the specification and is not intended to limit the scope of the present application. The scope of protection of the present application shall be defined by the appended claims.

### Definitions

As used herein, the term "epimerase" refers to an enzyme that catalyzes the change in configuration of one asymmetric carbon atom in a monosaccharide molecule (containing two or more asymmetric carbon atoms). In the present application, the epimerase refers to an enzyme that isomerizes aldohexoses. In the present application, the aldohexose is glucose or mannose. The epimerase described in the present application can convert glucose into mannose or convert mannose into glucose.

As used herein, "homology", "identity" and "similarity" refer to the sequence similarity between two nucleic acid molecules. The positions in each sequence can be compared to determine "homology", "identity" or "similarity", and the sequences can be aligned for comparison purposes. When equivalent positions in the compared sequences are occupied by the same base, the molecules are identical at that position; when equivalent positions are occupied by the same or similar amino acid (e.g., similar in spatial properties or charge properties) residues, the molecules may be referred to as homologous (similar) at that position. The expression of percent homology/similarity or identity is a function of the number of identical or similar amino acids at positions shared by the compared sequences. "Unrelated" or "non-homologous" sequences share less than 40% identity, preferably less than 25% identity, with the sequences of the present application. When comparing two sequences, the deletion of residues (amino acids or nucleic acids) or the presence of additional residues also reduces identity and homology/similarity. In specific embodiments, for two or more sequences or subsequences, when compared and aligned for maximum correspondence over a comparison window or specified region, as determined using the BLAST or BLAST 2.0 sequence comparison algorithm with default parameters described below or by manual alignment and visual inspection, for example provided online by the National Center for Biotechnology Information (NCBI), if their sequences are about 60%, or about 65%, 70%, 75%, 80%, 85%, 90%, 91%, 92%, 93%, 94%, 95%, 96%, 97%, 98%, 99% or more identical over the specified region, they may be considered substantially or significantly homologous, similar or identical. This definition also refers to or may be used for complements of test sequences. Thus, to the extent permitted by the context herein, for example, if a nucleotide sequence can be predicted to naturally exist in a DNA duplex, or can naturally exist in the form of one or both of the complementary strands, a nucleotide sequence complementary to a specified target sequence or variant thereof is itself considered "similar" to the target sequence, and when referring to "similar" nucleic acid sequences, the sequences includes single-stranded sequences, their complementary sequences, double-stranded chain complexes, sequences capable of encoding the same or similar polypeptide products, and any permissible variants of any of the foregoing. Cases where similarity must be limited to analysis of a single nucleic acid strand sequence may include, for example, detection and quantification of expression of a particular RNA sequence or coding sequence in a cell. This definition also includes sequences with deletions and/or additions, as well as sequences with substitutions. In embodiments, identity or similarity may be over a region of at least about 9, 10, 11, 12, 13, 14, 15, 16, 17, 18, 19, 10, 21, 22, 23, 24, 25 or more nucleotides in length, or over a region of more than about 10, 15, 20, 25, 30, 35, 40, 45, 50, 55, 60, 65, 70, 75, 80, 85, 90, 95 or more than about 100 nucleotides in length.

As used herein, the term "nucleotide" refers to naturally occurring nucleotides, as well as synthetic nucleotide analogs that can be recognized by cellular enzymes.

As used herein, the term "expression vector" refers to any naturally occurring or artificially constructed expression vector containing a nucleic acid molecule, wherein the nucleic acid molecule can be catalyzed by cellular transcription enzymes and/or translation enzymes. Exemplary expression vectors include: plasmids, viruses (including bacteriophages), cosmids, artificial chromosomes, or transposons, etc. In some embodiments, the expression vector is a plasmid.

As used herein, the term "host cell" refers to any biological cell that can be cultured in a culture medium and used to express a recombinant gene. These host cells may be eukaryotic or prokaryotic cells, microorganisms such as bacterial cells, or cells derived from a cell line (such as a mammalian immortalized cell line), etc. In some embodiments, the host cell is a prokaryotic cell, e.g., *Escherichia coli.*

As used herein, the term "recombinant" refers to a nucleic acid molecule or polypeptide that is located in a non-naturally occurring environment and is prepared through human intervention.

### Aldohexose-2-epimerase

The present application provides an aldohexose-2-epimerase, wherein the aldohexose-2-epimerase comprises a sequence having at least 90% identity to the sequence as set forth in any one of SEQ ID NOs: 1-5 or is a sequence having at least 90% identity to the sequence as set forth in any one of SEQ ID NOs: 1-5.

The aldohexose-2-epimerase comprising a sequence having at least 90% identity to the sequence set forth in any one of SEQ ID NOs: 1-5 , for example, can comprise a sequence having at least 90% identity, at least 91% identity, at least 92% identity, at least 93% identity, at least 94% identity, at least 95% identity, at least 96% identity, at least 97% identity, at least 98% identity, at least 99% identity or 100% identity to the sequence as set forth in any one of SEQ ID NOs: 1-5.

In the present application, the sequence of SEQ ID NO: 1 is as follows:

ENLTEWKRILAYWEKFSPDYQRGGFHGQVNYDNQPVLDASRSIILISRILWTFSLAYRHF
HRRRYLVLADRAYHYLYNHFRDTKNGGVYWSVTAAGVPLETRKQLYGHAFAIYGLSEYYA
ASKFKPALDFAQELFQTVDKHGYDAEKGGYFEAF

The sequence of SEQ ID NO: 2 is as follows:
GPSWEXVDDLILSZMPWNKSQN, wherein X is T or P or L, and Z is K or R.

The sequence of SEQ ID NO: 3 is as follows:

THLHIIEAFTNLYRVWPDALLKKRVVHLTDAFMEKLVSPETYRLRLFFDRDWQPKDETI
SYGHDIEASWLLWETAEVLNDHERSQKVKQLCIKMAEAACTGLGEDGALDYEFDPAANHR
NHERSWWVLAEQMVGFYNAYELTGESHYKDKSLKSWEFIKKYVMDTQ

The sequence of SEQ ID NO: 4 is as follows:
KGDWBGTVKPDLTPVRNAKOSFWKCPYHNSRACYE, wherein B is F or Y, and O is V or I.

The sequence of SEQ ID NO: 5 is as follows:

MDRFARENLTEWKRILAYWEKFSPDYQRGGFIIGQVNYDNQPVLDASRSIILISRILWTFS
LAYRHFHRRRYLVLADRAYHYLYNHFRDTKNGGVYWSVTAAGVPLETRKQLYGHAFAIYG
LSEYYAASKFKPALDFAQELFQTVDKHGYDAEKGGYFEAFGPSWETVDDLILSKMPWNKSQ
NTHLHIIEAFTNLYRVWPDALLKKRVVHLTDAFMEKLVSPETYRLRLFFDRDWQPKDETISY
GHDIEASWLLWETAEVLNDHERSQKVKQLCIKMAEAACTGLGEDGALDYEFDPAANHRNH
ERSWWVLAEQMVGFYNAYELTGESHYKDKSLKSWEFIKKYVMDTQKGDWFGTVKPDLTP
VRNAKVSFWKCPYHNSRACYEIVRRLEK

Those skilled in the art will also understand that the aldohexose-2-epimerase is not limited to the specific sequences listed above. The sequences of the mutants should encompass sequences that, compared to the sequence as set forth in any one of SEQ ID NOs: 1-5, comprise one, two, or more than three mutations but still have substantially the same function, as well as sequences that have 95%, 96%, 97%, 98%, or 99% sequence identity to the sequence as set forth in any one of SEQ ID NOs: 1-5.

In some embodiments, the aldohexose-2-epimerase comprises the sequence as set forth in any one of SEQ ID NOs: 1-5 or is the sequence as set forth in any one of SEQ ID NOs: 1-5. In some embodiments, the aldohexose is glucose or mannose, preferably D-glucose or D-mannose.

### Aldohexose-2-epimerase mutant

The present application provides an aldohexose-2-epimerase mutant, wherein the aldohexose-2-epimerase mutant comprises one or more mutations based on a reference sequence, and the amino acid sequence of the reference sequence is as set forth in SEQ ID NO: 5.

This sequence is a protein encoded by the gene Runsl_4586 derived from *Runella slithyformis*, named RsAE (*Runella slithyformis* aldohexose 2-epimerase), which is classified in the database as the N-acylglucosamine 2-epimerase (AGE) protein family.

In some embodiments, the amino acid sequence of the mutant comprises at least one an amino acid mutation at the positions corresponding to G354 and T355 of SEQ ID NO: 5, preferably comprises amino acid mutations at the positions corresponding to G354 and T355 of SEQ ID NO: 5.

In the present application, for the above-mentioned positions, numbering starts from the N-terminus. For example, G354 refers to an amino acid mutation occurring at the 354th amino acid from the N-terminus of SEQ ID NO: 5.

The term "corresponding to" has the meaning commonly understood by those of ordinary skill in the art. Specifically, "corresponding to" means that after two sequences are aligned for homology or sequence identity, a position in one sequence corresponds to a specified position in the other sequence.

In the present application, the glycine G at position 354 in SEQ ID NO: 5 can be mutated to glutamine Q, or the threonine T at position 355 can be mutated to glutamic acid E, or the glycine G at position 354 can be mutated to glutamine Q and the threonine T at position 355 can be mutated to glutamic acid E; preferably, the glycine G at position 354 is mutated to glutamine Q and the threonine T at position 355 is mutated to glutamic acid E.

Among them, the amino acid sequence of mutating glycine G at position 354 to glutamine Q is as set forth in SEQ ID NO: 6;
the amino acid sequence of mutating threonine T at position 355 to glutamic acid E is as set forth in SEQ ID NO: 7;
the amino acid sequence of mutating glycine G at position 354 to glutamine Q and threonine T at position 355 to glutamic acid E is as set forth in SEQ ID NO: 8.

The sequence of SEQ ID NO: 6 is as follows:

MDRFARENLTEWKRILAYWEKFSPDYQRGGFHGQVNYDNQPVLDASRSIILISRILWTFS
LAYRHFHRRRYLVLADRAYHYLYNHFRDTKNGGVYWSVTAAGVPLETRKQLYGHAFAIYG
LSEYYAASKFKPALDFAQELFQTVDKHGYDAEKGGYFEAFGPSWETVDDLILSKMPWNKSQ
NTHLHIIEAFTNLYRVWPDALLKKRVVHLTDAFMEKLVSPETYRLRLFFDRDWQPKDETISY
GHDIEASWLLWETAEVLNDHERSQKVKQLCIKMAEAACTGLGEDGALDYEFDPAANHRNH
ERSWWVLAEQMVGFYNAYELTGESHYKDKSLKSWEFIKKYVMDTQKGDWFQTVKPDLTP
VRNAKVSFWKCPYHNSRACYEIVRRLEK

The sequence of SEQ ID NO: 7 is as follows:

MDRFARENLTEWKRILAYWEKFSPDYQRGGFHGQVNYDNQPVLDASRSIILISRILWTFS
LAYRHFHRRRYLVLADRAYHYLYNHFRDTKNGGVYWSVTAAGVPLETRKQLYGHAFAIYG
LSEYYAASKFKPALDFAQELFQTVDKHGYDAEKGGYFEAFGPSWETVDDLILSKMPWNKSQ
NTHLHIIEAFTNLYRVWPDALLKKRVVHLTDAFMEKLVSPETYRLRLFFDRDWQPKDETISY
GHDIEASWLLWETAEVLNDHERSQKVKQLCIKMAEAACTGLGEDGALDYEFDPAANHRNH
ERSWWVLAEQMVGFYNAYELTGESHYKDKSLKSWEFIKKYVMDTQKGDWFGEVKPDLTP
VRNAKVSFWKCPYHNSRACYEIVRRLEK

The sequence of SEQ ID NO: 8 is as follows:

MDRFARENLTEWKRILAYWEKFSPDYQRGGFHGQVNYDNQPVLDASRSIILISRILWTFS
LAYRHFHRRRYLVLADRAYHYLYNHFRDTKNGGVYWSVTAAGVPLETRKQLYGHAFAIYG
LSEYYAASKFKPALDFAQELFQTVDKHGYDAEKGGYFEAFGPSWETVDDLILSKMPWNKSQ
NTHLIIIIEAFTNLYRVWPDALLKKRVVHLTDAFMEKLVSPETYRLRLFFDRDWQPKDETISY
GHDIEASWLLWETAEVLNDHERSQKVKQLCIKMAEAACTGLGEDGALDYEFDPAANHRNH
ERSWWVLAEQMVGFYNAYELTGESHYKDKSLKSWEFIKKYVMDTQKGDWFQEVKPDLTP
VRNAKVSFWKCPYHNSRACYEIVRRLEK

Those skilled in the art will also understand that the mutants are not limited to the specific sequences listed above. The sequences of the mutants should encompass sequences that, compared to the sequence as set forth in any one of SEQ ID NOs: 6-8, comprise one, two, or more than three nucleotide mutations but still have substantially the same function, as well as sequences that have 95%, 96%, 97%, 98%, or 99% sequence identity to the sequence as set forth in any one of SEQ ID NOs: 6-8.

The present application does not impose any restrictions on the method for preparing mutants, which can be mutated according to conventional methods in the art, for example, directed mutagenesis, random mutagenesis, or construction of synthetic oligonucleotides can be used, and then the mutated DNA sequence can be expressed in a host cell to obtain mutants with amino acid sequence substitutions, insertions and/or deletions. In the present application, based on the analysis of the 3D structure and function of the enzyme, site-directed mutagenesis is used for mutation.

The present application provides an aldohexose-2-epimerase mutant, comprising the amino acid sequence as set forth in any one of SEQ ID NOs: 6-8 or being the amino acid sequence as set forth in any one of SEQ ID NOs: 6-8. In some embodiments, the aldohexose is glucose or mannose.

### Nucleic acid molecule, expression vector and host cell

The present application provides a nucleic acid molecule, comprising a sequence encoding the aldohexose-2-epimerase according to any one of the above. In some embodiments, the nucleic acid molecule comprises a sequence as set forth in any one of SEQ ID NOs: 9-16 or a sequence having at least 90% identity to the sequence as set forth in any one of SEQ ID NOs: 9-16.

In some embodiments, the nucleotide sequence encoding the above-mentioned aldohexose-2-epimerase or a mutant thereof is codon-optimized. Typically, codon optimization involves balancing the percentage of selected codons with the abundance of published human transfer RNAs so that no one is overloaded or restricted. In some cases, this may be necessary because most amino acids are encoded by more than one codon, and codon usage varies among organisms. Differences in codon usage between the transfected gene and the host cell may affect protein expression and immunogenicity of the nucleic acid construct. Typically, for codon optimization, codons are selected to choose those that are balanced with human usage frequency. Typically, the redundancy of amino acid codons is such that different codons encode an amino acid. In some embodiments, when selecting codons for substitution, it may be desirable that the resulting mutation is a silent mutation so that the codon change does not affect the amino acid sequence. Typically, the last nucleotide of a codon can be left unchanged without affecting the amino acid sequence.

The sequence of SEQ ID NO: 9 is as follows:

GAAAACCTGACCGAGTGGAAACGTATCTTAGCTTACTGGGAAAAATTTCGCCCGAC
TATCAGCGGGGAGGCTTTCATGGCAGGTTAATTATGATAATCAGCCCGTATTAGATGCTT
CTCGTTCAATTATTTAATCAGTCGTATCTTATGGACTTTTCCCTGGCGTACCGACATTTC
CACCGCCGACGTTATCTGGTCCTTGCAGACAGAGCTTATCATTATTTGTATAATCATTTTCG
TGACACAAAAAACGGGGGTGTTTATTGGTCGGTTACGGCAGCAGGTGTACCGCTCGAAA
CCCGTAAACAACTTTATGGTCACGCTTTTGCCATCTACGGATTGAGTGAATATTACGCAGC
TTCTAAATTTAAACCTGCTTTGGATTTTGCGCAGGAATTGTTTCAGACCGTTGATAAACAC
GGATACGATGCTGAAAAAGGCGGATATTTTGAAGCCTTC

The sequence of SEQ ID NO: 10 is as follows:

GGTCCAAGTTGGGAAACCGTTGACGATCTGATTTTGAGTAAGATGCCTTGGAATAAA
TCCCAAAAC

The sequence of SEQ ID NO: 11 is as follows:

ACTCATTTGCATATCATAGAAGCGTTTACCAATTTGTACCGCGTTTGGCCTGATGCTCT
TCTTAAGAAACGGGTAGTTCATCTTACAGACGCGTTTATGGAAAATTGGTCAGTCCTGA
AACGTATCGTTTGCGCCTGTTCTTTGATCGAGACTGGCAACCGAAAGATGAAACCATTTC
TTACGGTCACGACATTGAAGCTTCCTGGCTGCTGTGGGAAACCGCAGAAGTACTCAATGA
TCATGAACGAAGTCAAAAAGTAAAGCAGTTGTGCATCAAAATGGCAGAAGCAGCCTGTA
CGGGATTGGGAGAAGACGGTGCGCTGGATTATGAATTTGACCCCGCTGCCAATCACCGCA
ATCACGAACGAAGTTGGTGGGTCTTGGCCGAACAAATGGTTGGATTTTACAATGCGTATG
AGCTTACGGGGGAATCACACTATAAAGACAAATCACTGAAAAGCTGGGAGTTTATCAAA
AAATACGTGATGGATACTCAA

The sequence of SEQ ID NO: 12 is as follows:

AAAGGCGATTGGTTTGGGACTGTAAAGCCTGATTTAACTCCTGTACGAAATGCAAAA
GTGAGTTTTTGGAAATGCCCTTACCATAACAGTCGTGCGTGCTATGAA

The sequence of SEQ ID NO: 13 is as follows:

GAAAACCTGACCGAGTGGAAACGTATCTTAGCTTACTGGGAAAAATTTTCGCCCGAC
TATCAGCGGGGAGGCTTTCATGGGCAGGTTAATTATGATAATCAGCCCGTATTAGATGCTT
CTCGTTCAATTATTTTAATCAGTCGTATCTTATGGACTTTTTCCCTGGCGTACCGACATTTC

CACCGCCGACGTTATCTGGTCCTTGCAGACAGAGCTTATCATTATTTGTATAATCATTTTCG
TGACACAAAAAACGGGGTGTTTATTGGTCGGTTACGGCAGCAGGTGTACCGCTCGAAA
CCCGTAAACAACTTTATGGTCACGCTTTTGCCATCTACGGATTGAGTGAATATTACGCAGC
TTCTAAATTTAAACCTGCTTTGGATTTTGCGCAGGAATTGTTTCAGACCGTTGATAAACAC
GGATACGATGCTGAAAAAGGCGGATATTTTGAAGCCTTCGGTCCAAGTTGGGAAACCGTT
GACGATCTGATTTTGAGTAAGATGCCTTGGAATAAATCCCAAAACACTCATTTGCATATCA
TAGAAGCGTTTACCAATTTGTACCGCGTTTGGCCTGATGCTCTTCTTAAGAAACGGGTAGT
TCATCTTACAGACGCGTTTATGGAAAAATTGGTCAGTCCTGAAACGTATCGTTTGCGCCTG
TTCTTTGATCGAGACTGGCAACCGAAAGATGAAACCATTTCTTACGGTCACGACATTGAA
GCTTCCTGGCTGCTGTGGGAAACCGCAGAAGTACTCAATGATCATGAACGAAGTCAAAA
AGTAAAGCAGTTGTGCATCAAAATGGCAGAAGCAGCCTGTACGGGATTGGGAGAAGACG
GTGCGCTGGATTATGAATTTGACCCCGCTGCCAATCACCGCAATCACGAACGAAGTTGGT
GGGTCTTGGCCGAACAAATGGTTGGATTTACAATGCGTATGAGCTTACGGGGGAATCAC
ACTATAAAGACAAATCACTGAAAAGCTGGGAGTTTATCAAAAAATACGTGATGGATACTC
AAAAAGGCGATTGGTTTGGGACTGTAAAGCCTGATTTAACTCCTGTACGAAATGCAAAAG
TGAGTTTTTGGAAATGCCCTTACCATAACAGTCGTGCGTGCTATGAA

The sequence of SEQ ID NO: 14 is as follows:

ATGGACCGTTTCGCCCGTGAAAACCTGACGGAATGGAAACGCATCCTGGCTTACTGG
GAGAAATTCAGCCCGGATTACCAGCGTGGTGGTTTCCACGGCCAGGTCAACTATGATAAC
CAGCCGGTTCTGGATGCTTCTCGTTCCATCATTCTGATCTCTCGTATTCTGTGGACTTTCTC
TCTGGCTTACCGTCATTTCCATCGTCGTCGTTACCTGGTTCTGGCGGATCGCGCGTATCACT
ACCTGTACAATCACTTCCGCGATACCAAAAACGGTGGTGTTTACTGGTCTGTGACGGCCG
CGGGTGTTCCGCTGGAAACCCGTAAACAACTGTACGGCCACGCATTTGCCATCTACGGCC
TGTCCGAATACTATGCAGCTTCCAAATTCAAACCGGCGCTGGATTTCGCACAAGAACTGT
TTCAGACTGTTGACAAACACGGCTACGATGCTGAGAAAGGTGGTTACTTTGAAGCGTTC
GGCCCGTCTTGGGAAACCGTCGATGATCTGATTCTGAGCAAAATGCCGTGGAACAAATCT
CAGAACACTCATCTGCACATCATCGAAGCCTTCACGAACCTGTATCGTGTTTGGCCGGAC
GCGCTGCTGAAAAAACGCGTGGTACACCTGACCGACGCCTTCATGGAAAAACTGGTGAG
CCCGGAAACCTATCGTCTGCGTCTGTTCTTCGATCGCGACTGGCAGCCGAAAGATGAAAC
GATTTCTTATGGCCACGACATCGAAGCCTCCTGGCTGCTGTGGAGACCGCTGAAGTACT

GAACGACCATGAACGTTCCCAGAAGGTGAAGCAGCTGTGCATTAAAATGGCGGAAGCGG
CTTGCACTGGCCTGGGTGAAGATGGTGCGCTGGACTACGAATTTGACCCGGCTGCTAACC
ACCGTAACCACGAACGTAGCTGGTGGGTGCTGGCGGAGCAGATGGTTGGCTTCTACAAC
GCTTACGAACTGACCGGTGAATCCCATTACAAAGACAAGTCCCTGAAATCCTGGAATTC
ATTAAGAAGTACGTCATGGATACTCAGAAAGGCGACTGGTTCCAGACCGTAAAACCGGAT
CTGACTCCGGTTCGCAACGCGAAAGTCTCTTTCTGGAAATGCCCGTACCATAACTCTCGT
GCTTGCTATGAAATTGTGCGTCGCCTGGAAAAA

The sequence of SEQ ID NO: 15 is as follows:

ATGGACCGTTTCGCCCGTGAAAACCTGACGGAATGGAAACGCATCCTGGCTTACTGG
GAGAAATTCAGCCCGGATTACCAGCGTGGTGGTTTCCACGGCCAGGTCAACTATGATAAC
CAGCCGGTTCTGGATGCTTCTCGTTCCATCATTCTGATCTCTCGTATTCTGTGGACTTTCTC
TCTGGCTTACCGTCATTTCCATCGTCGTCGTTACCTGGTTCTGGCGGATCGCGTATCACT
ACCTGTACAATCACTTCCGCGATACCAAAAACGGTGGTGTTTACTGGTCTGTGACGGCCG
CGGGTGTTCCGCTGGAAACCCGTAAACAACTGTACGGCCACGCATTTGCCATCTACGGCC
TGTCCGAATACTATGCAGCTTCCAAATTCAAACCGGCGCTGGATTTCGCACAAGAACTGT
TTCAGACTGTTGACAAACACGGCTACGATGCTGAGAAAGGTGGTTACTTTGAAGCGTTC
GGCCCGTCTTGGGAAACCGTCGATGATCTGATTCTGAGCAAAATGCCGTGGAACAAATCT
CAGAACACTCATCTGCACATCATCGAAGCCTTCACGAACCTGTATCGTGTTTGGCCGGAC
GCGCTGCTGAAAAAACGCGTGGTACACCTGACCGACGCCTTCATGGAAAAACTGGTGAG
CCCGGAAACCTATCGTCTGCGTCTGTTCTTCGATCGCGACTGGCAGCCGAAAGATGAAAC
GATTTCTTATGGCCACGACATCGAAGCCTCCTGGCTGCTGTGGGAGACCGCTGAAGTACT
GAACGACCATGAACGTTCCCAGAAGGTGAAGCAGCTGTGCATTAAAATGGCGGAAGCGG
CTTGCACTGGCCTGGGTGAAGATGGTGCGCTGGACTACGAATTTGACCCGGCTGCTAACC
ACCGTAACCACGAACGTAGCTGGTGGGTGCTGGCGGAGCAGATGGTTGGCTTCTACAAC
GCTTACGAACTGACCGGTGAATCCCATTACAAAGACAAGTCCCTGAAATCCTGGGAATTC
ATTAAGAAGTACGTCATGGATACTCAGAAAGGCGACTGGTTCGGTGAAGTAAAACCGGAT
CTGACTCCGGTTCGCAACGCGAAAGTCTCTTTCTGGAAATGCCCGTACCATAACTCTCGT
GCTTGCTATGAAATTGTGCGTCGCCTGGAAAAA

The sequence of SEQ ID NO: 16 is as follows:

ATGGACCGTTTCGCCCGTGAAAACCTGACGGAATGGAAACGCATCCTGGCTTACTGG

GAGAAATTCAGCCCGGATTACCAGCGTGGTGGTTTCCACGGCCAGGTCAACTATGATAAC
CAGCCGGTTCTGGATGCTTCTCGTTCCATCATTCTGATCTCTCGTATTCTGTGGACTTTCTC
TCTGGCTTACCGTCATTTCCATCGTCGTCGTTACCTGGTTCTGGCGGATCGCGCGTATCACT
ACCTGTACAATCACTTCCGCGATACCAAAAACGGTGGTGTTTACTGGTCTGTGACGGCCG
CGGGTGTTCCGCTGGAAACCCGTAAACAACTGTACGGCCACGCATTTGCCATCTACGGCC
TGTCCGAATACTATGCAGCTTCCAAATTCAAACCGGCGCTGGATTTCGCACAAGAACTGT
TTCAGACTGTTGACAAACACGGCTACGATGCTGAGAAAGGTGGTTACTTTGAAGCGTTC
GGCCCGTCTTGGGAAACCGTCGATGATCTGATTCTGAGCAAAATGCCGTGGAACAAATCT
CAGAACACTCATCTGCACATCATCGAAGCCTTCACGAACCTGTATCGTGTTTGGCCGGAC
GCGCTGCTGAAAAAACGCGTGGTACACCTGACCGACGCCTTCATGGAAAAACTGGTGAG
CCCGGAAACCTATCGTCTGCGTCTGTTCTTCGATCGCGACTGGCAGCCGAAAGATGAAAC
GATTTCTTATGGCCACGACATCGAAGCCTCCTGGCTGCTGTGGGAGACCGCTGAAGTACT
GAACGACCATGAACGTTCCCAGAAGGTGAAGCAGCTGTGCATTAAAATGGCGGAAGCGG
CTTGCACTGGCCTGGGTGAAGATGGTGCGCTGGACTACGAATTTGACCCGGCTGCTAACC
ACCGTAACCACGAACGTAGCTGGTGGGTGCTGGCGGAGCAGATGGTTGGCTTCTACAAC
GCTTACGAACTGACCGGTGAATCCCATTACAAAGACAAGTCCCTGAAATCCTGGGAATTC
ATTAAGAAGTACGTCATGGATACTCAGAAAGGCGACTGGTTCCAGGAAGTAAAACCGGA
TCTGACTCCGGTTCGCAACGCGAAAGTCTCTTTCTGGAAATGCCCGTACCATAACTCTCG
TGCTTGCTATGAAATTGTGCGTCGCCTGGAAAAA

The present application provides an expression vector, comprising the nucleic acid molecule described above. In some embodiments, the expression vector is a plasmid, cosmid, phage, or viral vector, preferably a plasmid or the like.

For example, the nucleic acid molecule encoding the above-mentioned aldohexose-2-epimerase or a mutant thereof can be cloned into a suitable expression vector, and the expression vector can be any suitable recombinant expression vector and can be used to transform or transfect any suitable host. Suitable vectors include those designed for propagation and amplification or for expression or for both, such as plasmids and viruses. In some embodiments, the expression vector is a plasmid.

In the present application, the expression vector may contain regulatory sequences (such as transcription and translation initiation and termination codons) that are specific to the type of host (e.g., bacteria, fungus, plant, or animal) into which the vector is to be introduced, as appropriate and considering whether the vector is DNA-based or RNA-based. The vector may also contain a non-natural promoter operably linked to the nucleotide sequence encoding the TCR. The promoter may be a non-viral promoter or a viral promoter, such as the cytomegalovirus (CMV) promoter, the SV40 promoter, the RSV promoter, and the promoter found in the long terminal repeat of murine stem cell virus, and other promoters known to those skilled in the art are also contemplated.

The present application provides a host cell, comprising the expression vector described above.

In the present application, the expression vector is transformed into a host cell for further expression or cloning in the host cell. In some embodiments, a method for preparing the aldohexose-2-epimerase or a mutant thereof is provided, the method comprising culturing the host cell provided above comprising the nucleic acid encoding the aldohexose-2-epimerase or a mutant thereof under conditions suitable for expressing the aldohexose-2-epimerase or a mutant thereof, and optionally recovering the aldohexose-2-epimerase or a mutant thereof from the host cell (or host cell culture medium). In some embodiments, the host cell is a eukaryotic cell, a prokaryotic cell, or a bacterial cell; preferably a prokaryotic cell; more preferably *Escherichia coli.*

The host cell refers to a cell into which exogenous nucleic acid has been introduced, including the progeny of such cells. Host cells include transformants and transformed cells, which include primary transformed cells and progeny derived therefrom, regardless of the number of passages. Progeny may not be completely identical to the parental cell in nucleic acid content, but may contain mutations.

### Use of the aldohexose-2-epimerase or a mutant thereof and method for converting aldohexose

The present application provides the use of the aldohexose-2-epimerase or a mutant thereof according to any one of the above in aldohexose conversion.

In the present application, the term "conversion" refers to the conversion between aldohexoses, for example, when the aldohexose is D-glucose, it can be converted into D-mannose, and when the aldohexose is D-mannose, it can be converted into D-glucose.

The present application provides a method for converting an aldohexose, comprising using the aldohexose-2-epimerase or a mutant thereof according to any one of the above to catalyze an aldohexose reaction, thereby converting the aldohexose. In some embodiments, the reaction temperature is 20-55°C, preferably 30-45°C; and/or
the reaction pH is 6.5-10, preferably 7.5-8.5; and/or
the reaction time is 0.5-10 hours, preferably 0.5-6 hours.

For example, the reaction temperature may be 20°C, 25°C, 30°C, 35°C, 40°C, 45°C, 50°C, or 55°C, etc.;
the reaction pH may be 6.5, 7, 7.5, 8, 8.5, 9, 9.5, or 10, etc.;
the reaction time may be 0.5 hours, 1 hour, 1.5 hours, 2 hours, 2.5 hours, 3 hours, 3.5 hours, 4 hours, 4.5 hours, 5 hours, 5.5 hours, 6 hours, 6.5 hours, 7 hours, 7.5 hours, 8 hours, 8.5 hours, 9 hours, 9.5 hours, or 10 hours, etc.

In some embodiments, the concentration of the aldohexose is 10-100 g/L.

For example, the concentration of the aldohexose can be 10 g/L, 20 g/L, 30 g/L, 40 g/L, 50 g/L, 60 g/L, 70 g/L, 80 g/L, 90 g/L, or 100 g/L, etc.

The aldohexose-2-epimerase or a mutant thereof described in the present application has high specificity. When the aldohexose is D-glucose, it can convert D-glucose into D-mannose; when the aldohexose is D-mannose, it can convert D-mannose into D-glucose. In addition, the aldohexose-2-epimerase has high enzyme activity. Through calculation and analysis, the specific enzyme activity of this enzyme in catalyzing the isomerization of D-glucose to D-mannose is about 6 enzyme activity units/mg enzyme, which can greatly improve the efficiency of converting D-glucose to produce D-mannose.

In addition, the aldohexose-2-epimerase mutant has high enzyme activity. Through calculation and analysis, 1 mg of the aldohexose-2-epimerase mutant (G354Q+T355E) contains about 13 enzyme activity units, and compared with the wild type, the specific enzyme activity is increased by about 2.2 times. Moreover, the mutant can increase the conversion rate of D-glucose to D-mannose, for example, the mutant (G354Q+T355E) can increase it by about 3.6 times.

### EXAMPLES

The present application provides a general and/or specific description of the materials and test methods used in the experiments. In the following examples, unless otherwise specified, % means wt%, i.e., weight percentage. The reagents or instruments used without indicating the manufacturers are all conventional reagent products that can be purchased commercially.

### Example 1: aldohexose-2-epimerase

### Example 1: Gene cloning, expression and preparation of RsAE (Runsl_4586)

The gene fragment Runsl_4586 from *R*. *slithyformis* (DSM 19594) is numbered AEI50906.1 in GenBank, which encodes a protein of 390 amino acids in full length (SEQ ID NO: 5). The Runsl_4586 gene fragment (SEQ ID NO: 10) was synthesized by entrusting Beijing Tsingke Biotechnology Co., Ltd. and inserted between the restriction sites NdeI and NotI of the pET22b plasmid. Finally, the expression plasmid was transformed into *Escherichia coli* BL21 to obtain recombinant *Escherichia coli.*

The recombinant *Escherichia coli* was inoculated into a test tube containing 5 mL of LB liquid medium and cultured overnight at 25°C and 150 rpm to obtain a seed culture. The next day, the seed culture was transferred at inoculation volume of 10% into a 500 mL shake flask containing 90 mL of fresh LB liquid medium, and cultured in a shaker at 37°C and 150 rpm. When the bacterial concentration OD₆₀₀ reached 0.6-0.8, the shaker temperature was adjusted to 28°C, IPTG was added to a final concentration of 0.5 mM, and induction expression for enzyme production was carried out at 28°C and 150 rpm for 5 hr.

The collected bacterial cells were resuspended in phosphate buffer (pH 7.8) and lysed by high pressure homogenization, then the supernatant was collected by centrifugation, and finally the aldohexose-2-epimerase RsAE (SEQ ID NO: 5) was obtained by purification through Ni-column affinity adsorption.

### Example 2: Identification of enzyme activity of RsAE

In a 1 mL reaction system (50 mM phosphate buffer (pH 7.8)), with a test substrate (as shown in Table 1) concentration of 10 g/L, 0.03 mg of RsAE obtained in Example 1 was added, and the reaction was carried out at 37°C for 60 min. After the reaction, the reaction was terminated in a boiling water bath for 10 min. The catalytic solution after termination of the reaction was centrifuged at 13000 rpm for 1 min, and the supernatant was taken to prepare HPLC test samples. High performance liquid chromatography was used to determine the amount of mannose produced in the reaction system, and the production status is shown in Table 1. The chromatographic conditions were as follows: using an Agilent 1260 high-performance liquid chromatograph, equipped with a Phenomenex MARS Mca 5u 300X 7.8 mm column, with a detector temperature of 35°C, column temperature of 80°C, and pure water as the mobile phase; the flow rate was 0.5 ml/min, and the injection volume was 10 µl.

**Table 1**

| Substrate | Reactivity | Remark |
|---|---|---|
| D-glucose | + | D-mannose generated |
| D-mannose | + | D-glucose generated |
| D-fructose | - | |
| D-galactose | - | |
| D-tagatose | - | |
| L-arabinose | - | |
| D-xylose | - | |
| L-rhamnose | - | |
| Lactose | - | |
| Sucrose | - | |
| Maltose | - | |
| N-acetyl-D-glucosamine | - | |

It can be seen from Table 1 that when the substrates catalyzed by RsAE are D-glucose and D-mannose, respectively, the reaction can generate the definite products D-mannose and D-glucose, respectively, but cannot catalyze other substrates, indicating that the enzyme described in the present application has specificity.

### Example 3: Determination of enzyme activity of RsAE in converting D-glucose to D-mannose

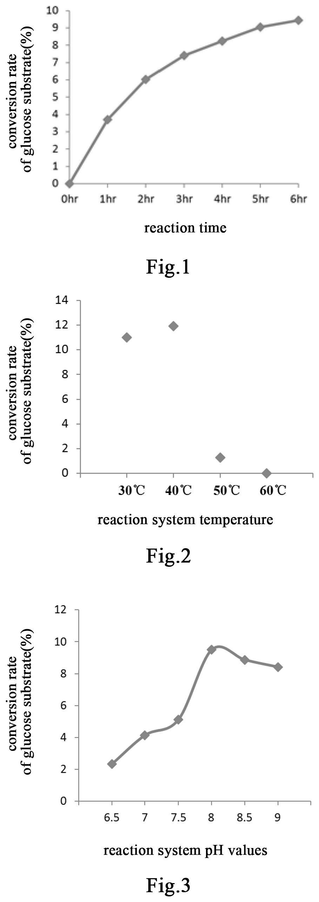

In a 1 mL reaction system (50 mM phosphate buffer (pH 7.8)), with a substrate glucose concentration of 10 g/L, 0.03 mg of RsAE prepared in Example 1 was added, and the reaction was carried out at 37°C for 0.5 hr, 1 hr, 2 hr, 3 hr, 4 hr, 5 hr and 6 hr, respectively. After the reaction, the reaction was terminated in a boiling water bath for 10 min. HPLC test sample preparation and high performance liquid chromatography conditions were the same as in Example 2. The reaction progress curve of RsAE in converting D-glucose to D-mannose is shown in Figure 1, wherein the enzyme activity is defined as: the amount of enzyme required to produce 1 µmol of mannose in 1 min at 37°C is 1 U.

From the relationship between glucose conversion rate and reaction time in Figure 1, and through calculation and analysis, it was obtained that 1 mg of RsAE contains about 6 enzyme activity units, i.e., 6 U/mg enzyme.

### Example 4: Study on the enzyme performance of RsAE in converting D-glucose to D-mannose

### (1) Enzyme kinetics study

In a 1 mL reaction system (50 mM phosphate buffer (pH 7.8)), with a substrate glucose concentration of 10 g/L~100 g/L, 0.03 mg of RsAE obtained in Example 1 was added respectively, and the reaction was carried out at 37°C for 60 min. After the reaction, the reaction was terminated in a boiling water bath for 10 min. HPLC test sample preparation and high performance liquid chromatography conditions were the same as in Example 2. The measured data were calculated and analyzed by the Lineweaver-Burk equation (double reciprocal plot method), and it was obtained that the *Km* of RsAE in converting D-glucose to D-mannose was about 440 mM, and the *Kcat* was about 5 s⁻¹.

### (2) Thermal stability of RsAE

In a 1 mL reaction system (50 mM phosphate buffer (pH 7.8)), with a substrate glucose concentration of 10 g/L, 0.03 mg of RsAE obtained in Example 1 was added, and the reaction was carried out at 30°C, 40°C, 50°C and 60°C for 120 min, respectively. After the reaction, the reaction was terminated in a boiling water bath for 10 min. HPLC test sample preparation and high performance liquid chromatography conditions were the same as in Example 2. The relationship between glucose substrate conversion rate and reaction system temperature was calculated and is shown in Figure 2. It can be seen that the stability of RsAE is better below 40°C.

### (3) Optimum pH of RsAE

In a 1 mL reaction system (50 mM phosphate buffer (pH 7.8)), with buffer pH values of 6.5, 7.0, 7.5, 8.0, 8.5 and 9.0, respectively, and a substrate glucose of 10 g/L, 0.03 mg of RsAE was added, and the reaction was carried out at 37°C for 120 min. After the reaction, the reaction was terminated in a boiling water bath for 10 min. HPLC test sample preparation and high performance liquid chromatography conditions were the same as in Example 2. The relationship between glucose substrate conversion rate and reaction system pH was calculated and is shown in Figure 3. The optimum pH of RsAE in converting D-glucose to D-mannose is around 8.0.

### Example 2: Mutants of aldohexose-2-epimerase

### Example 5: Preparation of gene cloning and expression of RsAE (Runsl_4586) wild type (SEQ ID NO: 5) and mutants

RsAE enzymes (wild type and mutants) were obtained according to the same method as in Example 1.

### Example 6: Identification and comparison of enzyme activity of RsAE wild type and mutants

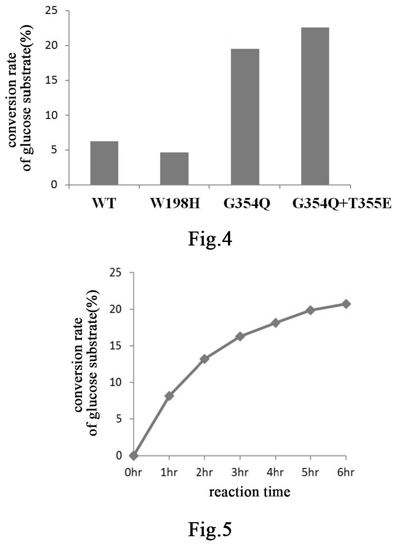

In a 1 mL reaction system (50 mM phosphate buffer (pH 7.8), with a substrate glucose concentration of 10 g/L, 0.15 mg of each of the enzymes prepared in Example 5 (RsAE wild type (WT), mutant (W198H), mutant (G354Q), and mutant (G354Q+T355E)) were added respectively, and the reaction was carried out at 37°C for 120 min. After the reaction, the reaction was terminated in a boiling water bath for 10 min. The catalytic solution after termination of the reaction was centrifuged at 13000 rpm for 1 min, and the supernatant was taken to prepare high performance liquid chromatography test samples, and the amount of mannose produced in the reaction system was measured. The chromatographic conditions of high performance liquid chromatography were as follows: using an Agilent 1260 high performance liquid chromatograph, equipped with a Phenomenex MARS Mca 5u 300X 7.8 mm chromatographic column, with a detector temperature 35°C, column temperature 80°C, and pure water the mobile phase; the flow rate was 0.5 mL/min, and the injection volume was 10 µL. The results are shown in Figure 4.

It can be seen from Figure 4 that under the above reaction conditions, the conversion rates of RsAE wild type (WT), mutant (W198H), mutant (G354Q), and mutant (G354Q+T355E) in converting D-glucose substrate to D-mannose were 6.3%, 4.8%, 19.5%, and 22.6%, respectively, indicating that the introduction of mutation W198H reduced the enzyme activity of RsAE in converting D-glucose substrate to D-mannose, while the introduction of mutations G354Q and T355E significantly increased the enzyme activity.

### Example 7: Determination of enzyme activity of RsAE mutant (G354Q+T355E) in converting D-glucose to D-mannose

In a 1 mL reaction system (50 mM phosphate buffer (pH 7.8)), with a substrate glucose concentration of 10 g/L, 0.03 mg of the RsAE mutant prepared in Example 5 was added, and the reaction was carried out at 37°C for 0.5 hr, 1 hr, 2 hr, 3 hr, 4 hr, 5 hr and 6 hr, respectively. After the reaction, the reaction was terminated in a boiling water bath for 10 min. HPLC test sample preparation and high performance liquid chromatography conditions were the same as in Example 6. The reaction progress curve of RsAE mutant (G354Q+T355E) in converting D-glucose to D-mannose is shown in Figure 5. The enzyme activity is defined as: the amount of enzyme required to produce 1 µmol of mannose in 1 min at 37°C is 1 U.

From Figure 5 and through calculation and analysis, 1 mg of RsAE mutant (G354Q+T355E) contains about 13 enzyme activity units, i.e., 13 U/mg enzyme.

### Example 8: Enzyme kinetics study of RsAE mutant (G354Q+T355E) in converting D-glucose to D-mannose

In a 1 mL reaction system (50 mM phosphate buffer (pH 7.8)), with substrate glucose concentrations of 10 g/L, 20 g/L, 40 g/L, 60 g/L, 80 g/L and 100 g/L, respectively, 0.03 mg of the RsAE mutant (G354Q+T355E) prepared in Example 5 was added respectively, and the reaction was carried out at 37°C for 60 min. After the reaction, the reaction was terminated in a boiling water bath for 10 min. HPLC test sample preparation and high performance liquid chromatography conditions were the same as in Example 6. The measured data were calculated and analyzed by the Lineweaver-Burk equation (double reciprocal plot method), and it was obtained that the *Km* of RsAE mutant (G354Q+T355E) in converting D-glucose to D-mannose was about 440 mM, and the *Kcat* was about 10 s⁻¹.

The above descriptions are only preferred embodiments of the present application and are not intended to limit the present application in other forms. Any person skilled in the art may utilize the technical content disclosed above to make changes or modifications to equivalent embodiments with equivalent changes. However, any simple changes, equivalent changes and modifications made to the above embodiments based on the technical essence of the present application without departing from the content of the technical solution of the present application still fall within the protection scope of the technical solution of the present application.

## Claims

1. An aldohexose-2-epimerase or a mutant thereof, wherein the aldohexose-2-epimerase comprises a sequence as set forth in any one of SEQ ID NOs: 1-5 or a sequence having at least 90% identity to the sequence as set forth in any one of SEQ ID NOs: 1-5; or
the aldohexose-2-epimerase mutant comprises one or more mutations based on a reference sequence, wherein the amino acid sequence of the reference sequence is as set forth in SEQ ID NO: 5.

2. The aldohexose-2-epimerase or a mutant thereof according to claim 1, wherein the amino acid sequence of the mutant comprises at least one amino acid mutation at the positions corresponding to G354 and T355 of SEQ ID NO: 5, preferably comprises amino acid mutations at the positions corresponding to G354 and T355 of SEQ ID NO: 5.

3. An aldohexose-2-epimerase mutant, comprising an amino acid sequence as set forth in any one of SEQ ID NOs: 6-8 or an amino acid sequence having at least 90% identity to the amino acid sequence as set forth in any one of SEQ ID NOs: 6-8.

4. The aldohexose-2-epimerase or a mutant thereof according to any one of claims 1-3, wherein the aldohexose is glucose or mannose.

5. A nucleic acid molecule, encoding the aldohexose-2-epimerase or a mutant thereof according to any one of claims 1-4.

6. The nucleic acid molecule according to claim 5, wherein the nucleic acid molecule comprises a sequence as set forth in any one of SEQ ID NOs: 9-16 or a sequence having at least 90% identity to the sequence as set forth in any one of SEQ ID NOs: 9-16.

7. An expression vector, comprising the nucleic acid molecule according to claim 5 or 6.

8. The expression vector according to claim 7, wherein the expression vector is a plasmid, a cosmid, a phage, or a viral vector; preferably a plasmid.

9. A host cell, comprising the expression vector according to claim 7 or 8;
preferably, the host cell is a eukaryotic cell, a prokaryotic cell, or a bacterial cell; preferably a prokaryotic cell; more preferably *Escherichia coli.*

10. Use of the aldohexose-2-epimerase or a mutant thereof according to any one of claims 1-4 in aldohexose conversion.

11. A method for converting an aldohexose, comprising using the aldohexose-2-epimerase or a mutant thereof according to any one of claims 1-4 to catalyze an aldohexose reaction, thereby converting the aldohexose.

12. The method according to claim 11, wherein the reaction temperature is 20-55°C, preferably 30-45°C; and/or
the reaction pH is 6.5-10, preferably 7.5-8.5; and/or
the reaction time is 0.5-10 hours, preferably 0.5-6 hours.
